# EUROPÄISCHE PATENTSCHRIFT

(11) **EP 0 489 790 B1**
(45) Veröffentlichungstag und Bekanntmachung des Hinweises auf die Patenterteilung: **10.03.1993**
(21) Anmeldenummer: 90912909.0
(22) Anmeldetag: 27.08.1990
(51) Int. Cl.: A61F 5/02

(54) **HYPEREXTENSIONSORTHESE MIT VERSCHIEBBARER STERNALPELOTTE**
HYPEREXTENSION ORTHESIS WITH MOVABLE FRONT PAD
ORTHESE HYPEREXTENSIBLE A PELOTE STERNALE DEPLA ABLE

(30) Priorität: 30.08.1989 DE 3928628
(43) Veröffentlichungstag der Anmeldung: 17.06.1992
(73) Patentinhaber: MUNNY, Kurt, D-51465 Bergisch Gladbach (DE)
(72) Erfinder: MUNNY, Kurt, D-51465 Bergisch Gladbach (DE)
(74) Vertreter: Sternagel, Hans-Günther, Dr.
(86) Internationale Anmeldenummer: EP9001428
(87) Internationale Veröffentlichungsnummer: WO9103216

(56) Entgegenhaltungen:
- US-A- 2 808 050
- US-A-39 453 76
- US-E- 315 64

## Beschreibung

Die Erfindung richtet sich auf eine verbesserte Hyperextensionsorthese, insbesondere eine solche, bei der der Abstand der Sternalpelotte zur Basisplatte während des Tragens sich automatisch an unterschieldliche Körperhalterungen anpaßt.

Bei allen bekannten Dreipunktstütz-Korsetts nach Bähler, sogenannten Hyperextensionsorthesen, sind die Pelotten starr angeordnet. Der Abstand der Pelotten von der Basisplatte wird beim Anpassen durch den Orthopädiefachmann durch Verkürzen der Brust- und Bauchstäbe angepaßt. Bei älteren Patienten treten, bedingt durch Haltungsfehler und beim Sitzen insofern Schwierigkeiten auf, daß sich durch die veränderte Körperhaltung die Sternalpelotte nach oben verschiebt und auf die Luftröhre drückt und Scheuerstellen hinterläßt.

Aus der Anzeige der Fa. Otto Bock: "Hyperextensionsorthese in Modulbauweise" in medizinisch-Orthopädische Technik, Heft 5, 1987 und aus US-E-31,564 sind gattungsgemäße Hyperextensionsorthesen - allerdings ohne Basisplatte - bekannt, die mit Hilfe von Langlöchern ein einfaches Anpassen der Position von Sternalpelotte und Symphysenpelotte an die Körpergröße des Patienten erlauben. Damit ist jedoch nur eine einmalige Anpassung möglich. Das eigentliche Problem einer dynamischen Anpassung der Position der Sternalpelotte an die jeweilige Körperhaltung des Patienten wird damit aber nicht gelöst.

Aufgabe der vorliegenden Erfindung ist es daher, eine konstruktive Gestaltung einer Hyperextensionsorthese zu schaffen, die diese Nachteile vermeidet und auch bei unterschiedlicher Körperhaltung des Trägers die richtige Position der Sternalpelotte sicherstellt.

Diese Aufgabe wird gelöst durch eine Hyperextensionsorthese mit einer Basisplatte, einem sich davon nach unten erstreckenden Bauchstab mit Symphysenpelotte, nach beiden Seiten erstreckenden Stegen mit Verschlußelementen und einem sich nach oben erstreckenden Bruststab, an dessen Ende ein Langloch zum Eingriff einer Schraube zum Befestigen einer Sternalpelotte vorhanden ist. Das Kennzeichnende der erfindungsgemäßen Konstruktion besteht darin, daß die Sternalpelotte eine Nut zur Aufnahme des Bruststabes und eine sich vom unteren Ende der Pelotte aus erstreckende langgestreckte Zunge aufweist, zum verschiebbaren Eingriff in eine am Bruststab in Abstand vom Langloch ausgebildete Gleithülse.

Die Schraube zum Befestigen der Sternalpelotte wird so gewählt, daß der Schraubenkopf nur fest am Bruststab anliegt, so daß ein Gleiten im Langloch ohne weiteres möglich ist.

Vorzugsweise ist die Nut zur Aufnahme des Bruststabes auf der dem Körper abgewandten Seite der Sternalpelotte ausgebildet und die Gleithülse auf der dem Körper zugewandten Seite des Bruststabes angeordnet. Bei der Erstanpassung der Hyperextensionsorthese wird die Länge des Bruststabes so gewählt, daß die Hyperextensionsorthese an der richtigen Körperstelle anliegt. Verkürzt sich nun durch Veränderung der Körperhaltung der Abstand zwischen der Sternalpelotte und der Basisplatte, schiebt sich infolge der festen Auflage der Sternalpelotte am Körper die Zunge oder der Gleiter tiefer in die Gleithülse des Bruststabes ein, so daß das Hochschieben der Sternalpelotte und der Druck auf die Luftröhre unterbleibt. Führt die Körperhaltung zu einem vergrößerten Abstand der Sternalpelotte von der Basisplatte rutscht der Gleiter aus der Gleithülse etwas weiter heraus. Diese konstruktive Gestaltung eines Bewegungsgleiters paßt den Abstand zwischen Sternalpelotte und Basisplatte automatisch an die Körperhaltung an, wobei der Druck der Sternalpelotte auf den Körper grundsätzlich erhalten bleibt. Die durch das Korsett erreichte Entlastung der Wirbelkörper von Lenden- und Brustwirbelsäule bleibt ebenfalls erhalten.

Die Gleithülse kann aus Kunststoff oder Metall ausgebildet sein. Geeignete Kunststoffe sind Thermoplasten mit guten Oberflächeneigenschaften, wie Polyamid, Polyolefine, Polyurethane, Polycarbonat. Geeignet sind sowohl Homopolymere als auch Copolymere.

Die Gleithülse hat vorzugsweise einen U-förmigen Querschnitt, so daß zwischen dem Bruststab und der Gleithülse ein Raum für die Aufnahme der Zunge entsteht.

Wenn die Gleithülse aus Kunststoff ist, kann diese durch Kleben, Nieten oder Verschrauben mit dem Bruststab verbunden werden. Wenn die Gleithülse aus Metall ist, kann diese durch Kleben, Nieten, Löten oder Schweißen mit dem Bruststab verbunden sein. Die Gleithülse kann auch aus dem Material des Bruststabes selbst herausgearbeitet sein, wenn das Material entsprechend dick gewählt wird. Um eine gleitfähige Verbindung zwischen der Zunge und dem Bruststab herzustellen, wird nach einer anderen Ausführungsform der Bruststab zur Ausbildung einer Gleithülse soweit umgebördelt, daß die umgebogenen Ränder die Zunge 9 zumindest teilweise überdecken. Bei einer solchen Ausführungsform ist es bevorzugt, den ggf. verbleibenden Spalt zwischen den umgebördelten Rändern des Brustbandes mit einer Kunststoffolie abzudecken. Wenn die Zunge und der Bruststab aus Metall sind, ist es bevorzugt, die Oberfläche der Gleithülse und/oder die Zunge und den Bruststab mit einer gleitfähigen Kunststoffschicht zu versehen. Ein dafür besonders bevorzugter Kunststoff ist Polytetrafluorethylen. Es können aber auch andere Kunststoffe für die Oberflächenbeschichtung verwendet werden.

Üblicherweise ist der Bruststab aus Metall. Grundsätzlich ist es auch möglich, den Bruststab aus mit Fasern verstärktem Kunststoff auszubilden. In diesem Falle ist es aus fertigungstechnischen Gründen vorteilhaft, die Gleithülse gleichzeitig mitauszubilden, beispielsweise durch Spritzgießen.

Die Erfindung wird anhand der Figuren noch näher erläutert.

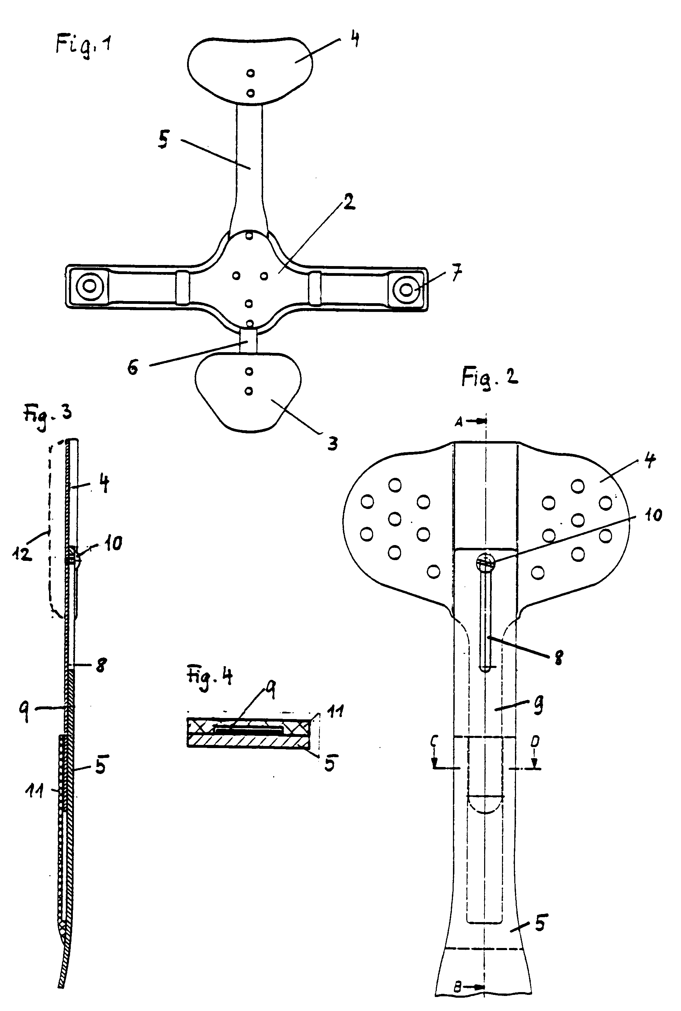

Figur 1 zeigt das Vorderteil einer Hyperextensionsorthese in einer schematischen Darstellung. Mit 2 ist die Basisplatte bezeichnet, von der aus sich nach unten der Bauchstab 6 erstreckt, an dessen Ende die Symphysenpelotte 3 befestigt ist. Von der Basisplatte 2 nach oben erstreckt sich der Bruststab 5 mit der an dessen Ende angeordneten Sternalpelotte 4. Von der Basisplatte 2 erstrecken sich nach jeder Seite Stege, an deren Enden jeweils Verschlußelemente 7 zur Verbindung mit dem nicht gezeigten Rückenteil vorhanden sind.

Figur 2 zeigt die erfindungsgemäße Ausbildung des Bruststabes 5 und der Sternalpelotte 4. Auf der dem Körper abgewandten Oberfläche der Sternalpelotte 4 ist eine Nut zur Aufnahme des Bruststabes 5 ausgebildet. Der Bruststab weist am Ende ein sich in Längsrichtung erstreckendes Langloch 8 auf. Die ovale Sternalpelotte 4 weist an ihrem unteren Ende eine langgestreckte Zunge als Gleiter zum Eingriff in die Gleithülse 11 auf, die zwischen der Hülse und dem Bruststab 5 geführt ist.

Die Verbindung der Sternalpelotte 4 mit dem Bruststab 5 wird durch die Schraube 10, die durch das Langloch 8 im Bruststab 5 in die Pelotte eingedreht ist, bewirkt. Der Schraubenkopf kann im Langloch 8 auf dem Bruststab 5 gleiten, gleichzeitig ändert sich die Einschubtiefe der Zunge 9 in die Gleithülse 11 im Bruststab.

Figur 3 ist ein Schnitt entlang der Linie A-B von Figur 2 und zeigt die Ausbildung der Führung für die Zunge 9, die als Gleiter 9 in der Gleithülse 11 ausgebildet ist. Die Sternalpelotte 4 ist dem Gleiter 9 ist durch diese konstruktive Gestaltung mit dem Bruststab 5 gleitfähig verbunden. Der Gleiter 9 schiebt sich in den Spalt zwischen der Gleithülse 11 und dem Bruststab 5 ein und ist gegen ein vollständiges Herausziehen durch die Schraube 10, die im Langloch 8 des Bruststabes gleiten kann, gesichert. Bevorzugt ist es, die dem Körper abgewandte Seite der Sternalpelotte 4 mit einer Polsterung 12 zu versehen. Als besonders vorteilhaft hat es sich erwiesen, die Polsterung 12 mit einem keilförmigen Querschnitt, d.h. am oberen Ende der Pelotte 4 dicker als am unteren Ende auszubilden.

Figur 4 ist ein Schnitt entlang der Linie C-D für Figur 2 und zeigt eine Ausführungsform der Gleithülse 11 an dem Bruststab 5 mit dem darin geführten Gleiter 9. Ein U-förmiges Teil 11 ist auf der dem Körper zugewandten Seite des Bruststabes 5 so angeordnet, daß der Gleiter 9 in den Schlitz zwischen dem Bruststab und den Schenkeln des U-Profils einschiebbar ist. Es ist jedoch auch grundsätzlich möglich, die Ränder des Bruststabes 5 so weit umzubördeln, daß sie über den Gleiter greifen, um eine Gleitführung auszubilden. Bei einer solchen Ausführungsform ist es vorteilhaft, die Führung vollflächig mit einer Schutzschicht abzudecken, um das Einklemmen von Fremdkörpern in die Führungsschiene zu vermeiden. Bruststab 5 und Gleiter 9 sind aus stabilem Material, bevorzugt aus Metall. Grundsätzlich ist es auch möglich, die Sternalpelotte mit einem Gleiter aus faserverstärktem Kunststoff herzustellen. Um ein dauerhaftes geräuscharmes Gleiten zu erreichen, ist es vorteilhaft, den Gleiter 9 oder die Oberfläche des Bruststabes 10 und die Gleithülse 11 mit einem gleitfähigen Kunststoff zu beschichten. Besonders vorteilhaft ist es, das U-förmige Teil 11 selbst aus einem gleitfähigen Kunststoff herzustellen.

Die feste Verbindung der Gleithülse 11 mit dem Bruststab 5 kann beispielsweise durch Kleben, Nieten, Schrauben oder Löten erfolgen.

Grundsätzlich ist es auch möglich, die Führung für den Gleiter 9 auf der dem Körper abgewandten Seite des Bruststabes 5 anzuordnen. Diese Ausführung ist jedoch nicht bevorzugt, weil die Gleithülse 11 dann stabiler ausgeführt sein muß, um die erforderliche Stützführung des Bruststabes 5 für die Sternalpelotte 4 zu gewährleisten.

## Patentansprüche

1. Hyperextensionsorthese mit einer Basisplatte (2), einem sich davon nach unten erstreckenden Bauchstab (6) mit Symphysenpelotte (3), sich nach beiden Seiten erstreckenden Stegen mit Verschlußelementen (7) und einem sich nach oben erstreckenden Bruststab (5), an dessen Ende ein Langloch (8) zum Eingriff einer Schraube (10) zum Befestigen einer Sternalpelotte (4) vorhanden ist,
**dadurch gekennzeichnet,**
daß die Sternalpelotte (4) eine Nut zur Aufnahme des Bruststabes (5) und eine sich vom unteren Ende der Sternalpelotte (4) aus erstreckende langgestreckte Zunge (9) aufweist, zum verschiebbaren Eingriff in eine am Bruststab (5) in Abstand vom Langloch (8) ausgebildete Gleithülse (11).

2. Hyperextensionsorthese nach Anspruch 1,
**dadurch gekennzeichnet,**
daß die Nut zur Aufnahme des Bruststabes (5) auf der dem Körper abgewandten Seite der Sternalpelotte (4) ausgebildet ist und die Gleithülse (11) auf der dem Körper zugewandten Seite des Bruststabes (5) angeordnet ist.

3. Hyperextensionsorthese nach Anspruch 1,
**dadurch gekennzeichnet,**
daß die Gleithülse einen U-förmigen Querschnitt aufweist.

4. Hyperextensionsorthese nach Anspruch 3,
**dadurch gekennzeichnet,**
daß die Gleithülse aus Kunststoff oder Metall ist.

5. Hyperextensionsorthese nach Anspruch 1,
**dadurch gekennzeichnet,**
daß der Seitenrand des Bruststabes zur Ausbildung einer Gleithülse (11), so weit umgebördelt ist, daß die umgebördelten Ränder die Zunge (9) zumindest teilweise überdecken.

## Claims

1. Hyperextension orthesis with a base plate (2), an abdominal rod (6) extending downwards therefrom with a symphysis pad (3), branches extending to both sides with locking elements (7), and an upwardly extending chest rod (5) at whose end an oblong hole (8) is provided for engagement of a screw (10) for securing a sternal pad (4), characterized in that the sternal pad (4) has a groove for receiving the chest rod (5) and an elongate tongue (9) extending out from the lower end of the sternal pad (4), for movable engagement in a slide sleeve (11) formed on the chest rod (5) at a distance from the oblong hole (8).

2. Hyperextension orthesis according to Claim 1, characterized in that the groove for receiving the chest rod (5) is arranged on the side of the sternal pad (4) directed away from the body, and the slide sleeve (11) is arranged on the side of the chest rod (5) directed toward the body.

3. Hyperextension orthesis according to Claim 1, characterized in that the slide sleeve has a U-shaped cross-section.

4. Hyperextension orthesis according to Claim 3, characterized in that the slide sleeve is made of plastic or metal.

5. Hyperextension orthesis according to Claim 1, characterized in that, in order to form a slide sleeve (11), the side edge of the chest rod is bent round to such an extent that the bent edges cover over the tongue (9) at least partially.

## Revendications

1. Orthèse hyperextensible comportant une plaque de base (2), une tige abdominale (6) à pelote symphysiale (3), s'étendant vers le bas à partir de la plaque de base, des branches, s'étendant latéralement, des deux côtés, à éléments de verrouillage (7) et une tige thoracique (5) s'étendant vers le haut, à l'extrémité de laquelle est présente une lumière oblongue (8) pour l'engagement d'une vis (10) pour la fixation d'une pelote sternale (4), caractérisée en ce que la pelote sternale (4) présente une rainure destinée à recevoir la tige thoracique (5) et une languette (9) allongée s'étendant à partir de l'extrémité inférieure de la pelote sternale (4) et destinée à s'engager, de manière déplaçable, dans une douille de coulissement (11) réalisée sur la tige thoracique (5), à distance de la lumière oblongue (8).

2. Orthèse hyperextensible suivant la revendication 1, caractérisée en ce que la rainure destinée à recevoir à tige thoracique (5) est réalisée sur la face de la pelote sternale (4) opposée au corps et que la douille de coulissement (11 ) est disposée sur la face de la tige thoracique (5) tournée vers le corps.

3. Orthèse hyperextensible suivant la revendication 1, caractérisée en ce que la douille de coulissement présente une section en forme de "U".

4. Orthèse hyperextensible suivant la revendication 3, caractérisée en ce que la douille de coulissement est en matière synthétique ou en métal.

5. Orthèse hyperextensible suivant la revendication 1, caractérisée en ce que le bord latéral de la tige thoracique est recourbé, pour la réalisation d'une douille de coulissement (11), à un point tel que les bords recourbés recouvrent la languette (9) au moins partiellement.
